(19) Europäisches Patentamt
European Patent Office
Office européen des brevets

(11) Veröffentlichungsnummer: **0 444 481 B1**

# (12) EUROPÄISCHE PATENTSCHRIFT

(45) Veröffentlichungstag der Patentschrift: **03.05.95**

(51) Int. Cl.6: **C07C 209/00**, C07C 209/60, C07C 211/02

(21) Anmeldenummer: **91102145.9**

(22) Anmeldetag: **15.02.91**

## (54) Verfahren zur Herstellung von Aminen.

(30) Priorität: **27.02.90 DE 4006112**

(43) Veröffentlichungstag der Anmeldung:
**04.09.91 Patentblatt 91/36**

(45) Bekanntmachung des Hinweises auf die Patenterteilung:
**03.05.95 Patentblatt 95/18**

(84) Benannte Vertragsstaaten:
**AT BE DE ES FR GB IT NL SE**

(56) Entgegenhaltungen:
**DE-A- 3 105 895**
**DE-A- 3 840 600**
**FR-A- 2 324 615**

**PATENT ABSTRACTS OF JAPAN, unexamined applications, Field C, Band 9, Nr. 78, 6.August 1985 THE PATENT OFFICE JAPANESE GOVERNMENT Seite 3 C 274**

(73) Patentinhaber: **HOECHST AKTIENGESELL-SCHAFT**

**D-65926 Frankfurt (DE)**

(72) Erfinder: **Kampmann, Detlef, Dr. Dpl.-Chem.**
**Friedhofstrasse 100**
**W-4630 Bochum (DE)**
Erfinder: **Weber, Jürgen,Dr. Dipl.-Chem**
**Bunsenstrasse 17**
**W-4200 Oberhausen 11 (DE)**
Erfinder: **Bahrmann, Helmut,Dr.Dipl.-Chem.**
**Rohstrasse 48**
**W-4231 Hamminkeln-Brünen (DE)**
Erfinder: **Kniep, Claus**
**Rosenstrasse 93**
**W-4200 Oberhausen 11 (DE)**

EP 0 444 481 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von Aminen durch katalytische Umsetzung von Olefinen mit Kohlenmonoxid, Wasserstoff und einem primären und/oder sekundären Amin unter Druck und erhöhter Temperatur in flüssiger Phase. Als Katalysatoren dienen Verbindungen von Elementen der Gruppe VIIIa des Periodensystems der Elemente.

Eine Beschreibung der auch als katalytische Aminomethylierung bezeichneten Reaktion von Olefinen mit Aminen, Kohlenmonoxid und Wasser in Gegenwart von Übergangsmetallkatalysatoren, die beispielsweise Rhodium, Ruthenium oder Iridium enthalten, ist F. Jachimowicz und J. W. Raksis, J. Org. Chem. 1982, 47, Seiten 445 bis 447 zu entnehmen. Dieses Verfahren liefert Amine in recht unterschiedlichen Ausbeuten und erfordert reines Kohlenmonoxid als Reaktant.

Die GB 2 113 210 A betrifft die Herstellung tertiärer Amine durch Umsetzung langkettiger Olefine mit Kohlenmonoxid, Wasserstoff und einem primären oder sekundären Amin in Gegenwart eines Rhodium- oder Rutheniumkatalysators, insbesondere $RhCl_3 \cdot 3H_2O$ und $RuCl_3 \cdot 3H_2O$. Als Lösungsmittel wird ein gegebenenfalls mit Wasser versetzter, ein- oder mehrwertiger Alkohol verwendet. Nach Umsetzung wird die den Alkohol enthaltende Lösungsmittelphase vom Reaktionsprodukt durch Phasenseparation abgetrennt, und der größtenteils im Lösungsmittel gelöste Katalysator zurückgewonnen. Setzt man den zurückgewonnenen Katalysator erneut in die Reaktion ein, so sinkt die Aminausbeute bereits nach wenigen Wiedereinsätzen deutlich ab. Da das Reaktionsgemisch den teuren Katalysator auch nach Phasentrennung noch in nicht vernachlässigbarer Menge enthält, läßt die durch wiederholte Separation der Lösungsmittelphase herbeigeführte Rückgewinnung des Katalysators und ihr Wiedereinsatz als Katalysator zu wünschen übrig. Ferner befindet sich ein Teil des Reaktionsproduktes in der Lösungsmittelphase und muß in einem gesonderten Arbeitsschritt abgetrennt werden.

Es besteht daher ein Bedarf nach einem Verfahren zur Herstellung von Aminen, das die vorstehend genannten Nachteile vermeidet. Diese Aufgabe wird gelöst durch ein Verfahren zur Herstellung von Aminen durch Umsetzung von Olefinen mit Kohlenmonoxid, Wasserstoff und einem primären und/oder sekundären Amin in flüssiger Phase in Gegenwart von Rhodium und Arylphosphanen bei erhöhter Temperatur und unter Druck, dadurch gekennzeichnet, daß als Arylphosphane Salze der allgemeinen Formel

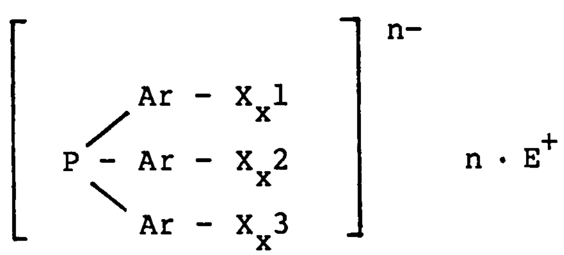

gelöst in Wasser eingesetzt werden, wobei Ar für einen Arylrest und X für eine Sulfonsäuregruppe steht, $x^1$, $x^2$, $x^3$ 0 oder 1 bedeuten mit der Maßgabe, daß mindestens eine Zahl $x^1$, $x^2$ oder $x^3$ 1 ist, E ein Alkalimetallatom, $NH_4^+$ oder ein quartäres Ammoniumion der allgemeinen Formel

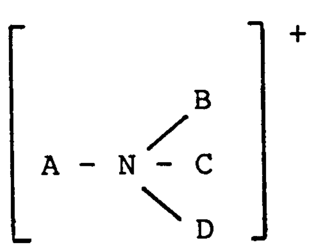

ist, in der A für einen Alkylrest mit 6 bis 20 Kohlenstoffatomen steht und B, C, D geradkettige oder verzweigte Alkylreste mit 1 bis 4 Kohlenstoffatomen sind und n eine ganze Zahl zwischen 1 und 3 ist.

Man führt die Umsetzung bei 100 bis 160, insbesondere 110 bis 150, bevorzugt bei 120 bis 140 °C und bei 4 bis 20, insbesondere 8 bis 18, bevorzugt 10 bis 16 MPa durch.

Es hat sich überraschenderweise gezeigt, daß es nicht erforderlich ist, ausschließlich quartäre Ammoniumsalze sulfonierter Triarylphosphane als Komplexliganden für das Rhodium zu verwenden, um auch höhere Olefine mit sehr gutem Erfolg durch das erfindungsgemäße Verfahren in die entsprechenden Amine zu überführen. Auch Gemische aus Alkali- oder Ammoniumsalzen und relativ geringen Mengen quartäre Ammoniumsalze der vorstehend aufgeführten allgemeinen Formel sind als Komplexliganden für das Rhodium geeignet.

Nach einer bevorzugten Ausführungsform des erfindungsgemäßen Verfahrens enthält das Gemisch der wasserlöslichen Phosphane 1 bis 40, insbesondere 10 bis 35 und vorzugsweise 15 bis 33 Mol-% (bezogen auf das Phosphangemisch) quartäre Ammoniumsalze.

Zu den Alkalisalzen der wasserlöslichen, sulfonierten Triarylphosphane gehören auch die Ammoniumverbindungen, also Salze, die das Kation $NH_4^+$ enthalten. Besonders zweckmäßig ist es, mit Natrium und/oder Kaliumsalzen zu arbeiten.

Die erfindungsgemäß eingesetzten quartären Ammoniumverbindungen zeichnen sich dadurch aus, daß sie einen kohlenstoffreichen Alkylrest A und 3 kurzkettige Alkylreste B, C und D enthalten. Sowohl der kohlenstoffreichere Rest als auch die kurzkettigen Alkylreste können unverzweigt, gegebenenfalls aber auch verzweigt sein. Bevorzugt werden quartäre Ammoniumionen, deren kohlenstoffreicher Rest ein geradkettiger Alkylrest mit 10 bis 18 und insbesondere mit 12 bis 16 Kohlenstoffatomen ist. Kurzkettige Alkylreste sind vorzugsweise Methyl- und/oder Ethylreste.

Als wasserlösliche Phosphane der oben wiedergegebenen allgemeinen Formel werden gemäß der neuen Arbeitsweise insbesondere Verbindungen eingesetzt, in denen Ar ein Phenyl- oder Naphthylrest, insbesondere ein Phenylrest und die Summe von $x^1$, $x^2$ und $x^3$ 2 oder 3 ist.

Beispiele für wasserlösliche Phosphane, die sich zur Durchführung des neuen Verfahrens eignen, sind Natrium- und/oder Kalium-Triphenylphosphan-trisulfonate und Triphenylphosphan-disulfonate sowie Tetraakylammoniumsalze der genannten Triphenylphosphansulfonate mit folgenden Kationen: Trimethylcetylammonium, Trimethyldodecylammonium, Trimethyltetradecylammonium, Trimethylhexadecylammonium, Dodecylethyldimethylammonium.

Zur Herstellung der in dem beanspruchten Verfahren verwendeten Phosphane geht man von sulfonierten Triarylphosphanen aus, die durch Behandlung von Triarylphosphanen mit Oleum erhalten werden. Durch Variation der Reaktionsbedingungen, insbesondere der Reaktionszeit, der Reaktionstemperatur und des Verhältnisses von Triarylphosphan zu Schwefeltrioxid lassen sich bevorzugt mono-, di- oder trisulfonierte Arylphosphane herstellen.

Zweckmäßig gewinnt man aus dem Sulfonierungsprodukt zunächst in Wasser unlösliche, in organischen Lösungsmitteln jedoch lösliche Aminsalze. Sie werden anschließend durch Behandlung mit einem quartären Ammoniumhydroxid in das gewünschte Onium-Salz des sulfonierten Triarylphosphans überführt.

Die Umsetzung der Olefine mit Wasserstoff, Kohlenmonoxid und Amin nach dem neuen Verfahren erfolgt bei erhöhter Temperatur und gesteigertem Druck.

Der Katalysator kann dem Reaktionssystem präformiert zugesetzt werden. Mit gleich gutem Erfolg läßt er sich aber auch aus den Komponenten Rhodium oder Rhodiumverbindung und der wäßrigen Lösung der Salze der sulfonierten Triarylphosphane unter Reaktionsbedingungen im Reaktionsgemisch, also in Gegenwart von Synthesegas, Amin und Olefin herstellen. Außer metallischem Rhodium in feinverteilter Form können als Rhodiumquelle wasserlösliche Rhodiumsalze wie Rhodiumchlorid, Rhodiumsulfat, Rhodiumacetat oder in organischen Medien lösliche Verbindungen wie Rhodium-2-ethylhexanoat oder unlösliche Verbindungen wie Rhodiumoxide eingesetzt werden.

Die Rhodiumkonzentration in der wäßrigen Katalysatorlösung beträgt 10 bis 2000 Gew.-ppm, vorzugsweise 50 bis 800 Gew.-ppm. bezogen auf die Lösung. Das quartäre Ammoniumsalz des sulfonierten Phosphans wird in einer solchen Menge eingesetzt, daß auf 1 Grammatom Rhodium 2 bis 300 Mol, vorzugsweise 10 bis 100 Mol Phosphanverbindung kommen.

Der pH-Wert der wäßrigen Katalysatorlösung soll nicht unter 2 liegen. Allgemein stellt man einen pH-Wert von 2 bis 13, vorzugsweise 5 bis 7, ein.

Die Zusammensetzung des Synthesegases, d.h. das Verhältnis von Kohlenmonoxid zu Wasserstoff, kann in weiten Grenzen variiert werden. Im allgemeinen setzt man ein Synthesegas ein, in dem das Volumverhältnis von Kohlenmonoxid zu Wasserstoff 1 : 1 bis 1 : 2,5 beträgt oder von diesem Wert nur wenig abweicht. Je Mol Olefin verwendet man 1 bis 12, insbesondere 1,2 bis 10, bevorzugt 1,5 bis 8 Mol CO und 2 bis 24, insbesondere 2,4 bis 20, bevorzugt 3 bis 16 Mol $H_2$. Es hat sich bewährt mit einem Überschuß von 10 bis 200, insbesondere 25 bis 120 Mol % CO und 20 bis 400, insbesondere 50 bis 240 Mol % $H_2$ bezogen auf Mol Olefin zu arbeiten.

Das für die Reaktion benötigte primäre und/oder sekundäre Amin setzt man in einer Menge von 1 bis 3, insbesondere 1 bis 2, bevorzugt 1 bis 1,5 Mol je Mol umzusetzendes Olefin ein. Geeignete Amine sind

aliphatische, geradkettige und/oder verzweigte primäre und/oder sekundäre Amine mit 1 bis 24, insbesondere 2 bis 12, bevorzugt 2 bis 8 Kohlenstoffatomen. Besonders bewährt haben sich Amine der Formel $R_1R_2NH$, worin $R_1$ und $R_2$ gleich oder verschieden sind und jeweils 1 bis 4 Kohlenstoffatome enthalten, insbesondere Dimethyl-, Diethyl- und Methylethylamin. Als primäre Amine lassen sich Methyl-, Ethyl- und Propylamin, insbesondere Methyl- und Ethylamin einsetzen.

Die Umsetzung kann sowohl absatzweise als auch kontinuierlich durchgeführt werden.

Nach Beendigung der Umsetzung läßt man das Reaktionsgemisch erforderlichenfalls abkühlen und trennt die untere, wäßrige Phase, die das aus Rhodium und Salzen sulfonierter Triarylphosphane bestehende Katalysatorsystem enthält, von der oberen organischen Phase ab.

Ein besonderer Vorteil des erfindungsgemäßen Verfahrens besteht darin, daß die abgetrennte wäßrige Katalysatorlösung mit sehr gutem Erfolg unmittelbar wieder in die Reaktion eingesetzt werden kann. Die Wirksamkeit der gebrauchten, wäßrigen Katalysatorphase nimmt erst nach mehrfachem Wiedereinsatz allmählich ab. Will man die Lebensdauer der gebrauchten wäßrigen Katalysatorlösung zusätzlich verbessern, so empfiehlt sich ein geringfügiger Zusatz von frischem Katalysator, um sowohl Aktivität als auch Selektivität der gebrauchten Katalysatorphase zu steigern.

Bei kontinuierlicher Arbeitsweise kann man diesen Vorteil des erfindungsgemäßen Verfahrens besonders einfach nutzen, indem man das aus organischem Produkt und wäßriger Katalysatorlösung bestehende Gemisch direkt einem Phasentrenngefäß zuleitet, die wäßrige Phase kontinuierlich abtrennt und gegebenenfalls nach Zusatz einer entsprechenden Menge frischen Katalysators in die Reaktionszone zurückführt. Diese Kreislaufführung wirkt sich besonders schonend aus und gewährleistet eine erhöhte Lebensdauer der gebrauchten Katalysatorlösung.

Die durch das Separieren der organischen Wertprodukte verursachten Katalysatorverluste sind relativ gering. Sie betragen pro Wiedereinsatz etwa 0,1 bis 1 Gew.-% Rh.

Das erfindungsgemäße Verfahren wird mit Erfolg bei der Umsetzung von geradkettigen oder verzweigten Olefinen mit 2 bis 24, insbesondere mit 8 bis 20, bevorzugt 12 bis 18 Kohlenstoffatomen angewandt. Die Doppelbindung in diesen Olefinen sollte bevorzugt endständig sein.

Geeignete Olefine sind n-Hexen-1, n-Hepten-1, n-Octen-1, n-Nonen-1, n-Decen-1, n-Dodecen-1, n-Tetradecen-1, n-Hexadecen-1, insbesondere n-Decen-1, n-Dodecen-1, n-Tetradecen-1.

Die nachfolgenden Beispiele erläutern die Erfindung näher, ohne sie auf die beschriebenen Ausführungsformen zu beschränken.

## Experimenteller Teil

a) Herstellung der Katalysatorlösung

In einem mit Tauchstutzen versehenen Autoklaven werden eine wäßrige Lösung, die die Na-Salze der Triphenylphosphan-m-disulfonsäure(TPPDS-Na) und der Triphenylphosphan-m-trisulfonsäure (TPPTS-Na) im Verhältnis 1 : (14 bis 15) und ein Oniumsalz im Verhältnis Oniumsalz zu Natriumsalzen von etwa 1 : 2 enthält und Rh-acetat entsprechend 200 ppm Rh vorgelegt. Das Phosphor/Rhodium Verhältnis beträgt 300 : 1.

Anschließend wird auf diese Lösung Synthesegas ($CO/H_2$ = 1 : 1) bis zu einem Druck von 10 MPa aufgepreßt. Man erhitzt unter Rühren auf 125 °C und behandelt die wäßrige Lösung 3 Stunden mit Synthesegas. Man kühlt auf etwa 30 °C, stellt die Rührung ab und drückt, nach einer Absetzzeit von etwa 15 Minuten,überschüssige Lösung über den Tauchstutzen heraus. Die restliche Katalysatorlösung verbleibt im Autoklaven.

b) Beispiele 1 bis 10

Zu 400 g der vorstehend beschriebenen Katalysatorlösung, die als Oniumsalz das n-Tetradecyltrimethylammoniumsalz enthält, werden 118 g (≙ 0,7 mol) Dodecen und 50 g (1,11 mol) Dimethylamin eingepumpt. Die Umsetzung erfolgt unter Rühren bei 140 °C und 10 MPa, wobei Synthesegas entsprechend seinem Verbrauch ergänzt wird. Nach etwa 4 Stunden ist die Aufnahme von Synthesegas beendet. Man läßt noch 1 Stunde nachreagieren, kühlt ab und drückt, nach einer Absetzzeit von 15 Minuten,die organische Phase über den Tauchstutzen heraus. Im Anschluß hieran werden erneut die vorstehend angegebenen Mengen n-Dodecen und Dimethylamin zugepumpt und die Umsetzung wie zuvor beschrieben durchgeführt.

Die jeweils anfallende organische Phase wird gewogen und gaschromatografisch untersucht. Die daraus resultierenden Ergebnisse sind der nachfolgenden Tabelle 1 zu entnehmen.

Tabelle 1

| Beispiel[1)] | | 1 | 2 | 3 | 4 | 5 | 6 | 7 | 8 | 9 | 10 |
|---|---|---|---|---|---|---|---|---|---|---|---|
| Umsatz[2)] | (%) | 94,5 | 94 | 90,25 | 87,97 | 81,59 | 81,66 | 79,39 | 77,01 | 81,69 | 75,6 |
| Selektivität[2)] | (%) | 78 | 84,9 | 91,2 | 92,8 | 94,3 | 93,5 | 93,5 | 93,5 | 92,1 | 94,2 |
| n : iso- | (n) | 58 | 65 | 70 | 71 | 72 | 73 | 71 | 70 | 71 | 72 |
| Verhältnis[3)] | (iso) | 42 | 35 | 30 | 29 | 28 | 27 | 29 | 30 | 29 | 28 |
| Ausbeute[2)] | (%) | 73,5 | 79,8 | 82,4 | 81,7 | 77,0 | 76,4 | 74,2 | 72,0 | 75,2 | 71,2 |

1) Beispiele 2 bis 10 durchgeführt mit wiedereingesetzer Katalysatorlösung aus Beispiel 1

2) bezogen auf eingesetztes Olefin

3) Verhältnis N,N-Dimethyl-n-tridecylamin : N,N-Dimethyl-2-methyldodecylamin

## Patentansprüche

1. Verfahren zur Herstellung von Aminen durch Umsetzung von Olefinen mit Kohlenmonoxid, Wasserstoff und einem primären und/oder sekundären Amin in flüssiger Phase in Gegenwart von Rhodium und

Arylphosphanen bei erhöhter Temperatur und unter Druck, dadurch gekennzeichnet, daß als Arylphosphane Salze der allgemeinen Formel

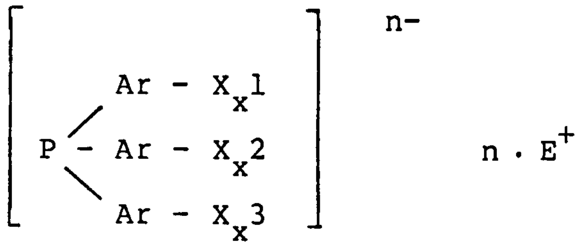

gelöst in Wasser eingesetzt werden, wobei Ar für einen Arylrest und X für eine Sulfonsäuregruppe steht, $x^1$, $x^2$, $x^3$ 0 oder 1 bedeuten mit der Maßgabe, daß mindestens eine Zahl $x^1$, $x^2$ oder $x^3$ 1 ist, E ein Alkalimetallatom, $NH_4^+$ oder ein quartäres Ammoniumion der allgemeinen Formel

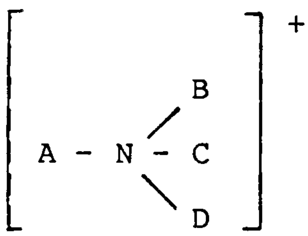

ist, in der A für einen Alkylrest mit 6 bis 20 Kohlenstoffatomen steht und B, C, D geradkettige oder verzweigte Alkylreste mit 1 bis 4 Kohlenstoffatomen sind und n eine ganze Zahl zwischen 1 und 3 ist.

**2.** Verfahren nach Anspruch 1, dadurch gekennzeichnet, daß die Umsetzung bei 100 bis 160, insbesondere 110 bis 150, bevorzugt 120 bis 140 °C und bei 4 bis 20, insbesondere 8 bis 18, bevorzugt 10 bis 16 MPa durchgeführt wird.

**3.** Verfahren nach Anspruch 1 oder 2, dadurch gekennzeichnet, daß als Arylphosphane ein Gemisch aus Alkali- oder Ammoniumsalzen und quartären Ammoniumsalzen der allgemeinen Formel

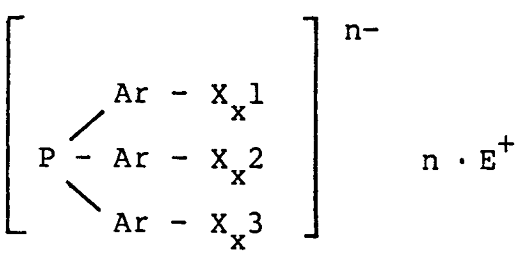

eingesetzt wird.

**4.** Verfahren nach einem oder mehreren der Ansprüche 1 bis 3, dadurch gekennzeichnet, daß das Gemisch der Arylphosphane 1 bis 40, insbesondere 10 bis 35 und vorzugsweise 15 bis 33 Mol-% (bezogen auf das Phosphangemisch) quartäre Ammoniumsalze enthält.

**5.** Verfahren nach einem oder mehreren der Ansprüche 1 bis 4, dadurch gekennzeichnet, daß die Alkalisalze der Arylphosphane Natrium und/oder Kalium als Kation enthalten.

**6.** Verfahren nach einem oder mehreren der Ansprüche 1 bis 5, dadurch gekennzeichnet, daß in der allgemeinen Formel des quartären Ammoniumions A einen Alkylrest mit 10 bis 18, insbesondere 12 bis

16 Kohlenstoffatomen bedeutet.

7. Verfahren nach einem oder mehreren der Ansprüche 1 bis 6, dadurch gekennzeichnet, daß in der allgemeinen Formel des quartären Ammoniumions B, C und D einen Methyl- und/oder Ethylrest bedeuten.

8. Verfahren nach Anspruch 6 und 7, dadurch gekennzeichnet, daß das quartäre Ammoniumion das Trimethyldodecylammoniumion, das Trimethyltetradecylammoniumion oder das Trimethylhexadecylammoniumion ist.

9. Verfahren nach einem oder mehreren der Ansprüche 1 bis 8, dadurch gekennzeichnet, daß die wäßrige, Rhodium und Arylphosphane enthaltende Katalysatorlösung einen pH-Wert von 5 bis 7 aufweist.

10. Verfahren nach einem oder mehreren der Ansprüche 1 bis 9, dadurch gekennzeichnet, daß die Olefine 2 bis 24, insbesondere 8 bis 20, bevorzugt 12 bis 18 Kohlenstoffatome enthalten.

## Claims

1. A process for the preparation of amines by reaction of olefins with carbon monoxide, hydrogen and a primary and/or secondary amine in liquid phase in the presence of rhodium and arylphosphanes at elevated temperature and under pressure, which comprises using salts of the general formula

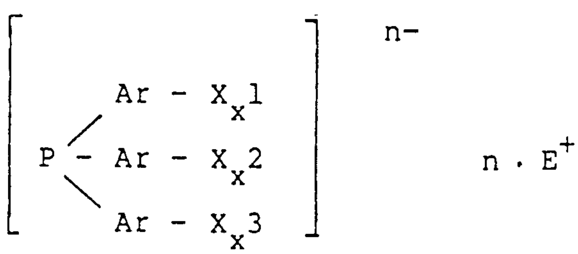

dissolved in water as arylphosphanes, in which Ar is an aryl radical and X a sulfo group, $x^1$, $x^2$, $x^3$ are 0 or 1, with the proviso that at least one number $x^1$, $x^2$ or $x^3$ is 1, E is an alkali metal atom, $NH_4^+$ or a quaternary ammonium ion of the general formula

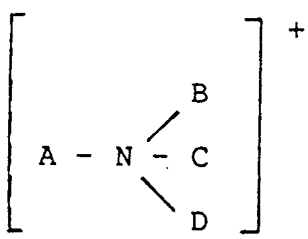

in which A is an alkyl radical having 6 to 20 carbon atoms and B, C, D are straight-chain or branched alkyl radicals having 1 to 4 carbon atoms and n is an integer between 1 and 3.

2. The process as claimed in claim 1, wherein the reaction is carried out at 100 to 160, in particular 110 to 150, preferably 120 to 140 °C and at 4 to 20, in particular 8 to 18, preferably 10 to 16, MPa.

3. The process as claimed in claim 1 or 2, wherein the arylphosphanes used are a mixture of alkali metal salts or ammonium salts and quaternary ammonium salts of the general formula

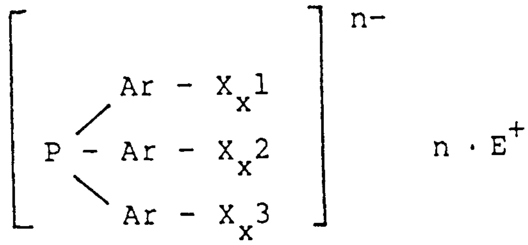

**4.** The process as claimed in one or more of claims 1 to 3, wherein the mixture of arylphosphanes contains 1 to 40, in particular 10 to 35, and preferably 15 to 33, mol% (relative to the phosphane mixture) of quaternary ammonium salts.

**5.** The process as claimed in one or more of claims 1 to 4, wherein the alkali metal salts of the arylphosphane contain sodium and/or potassium as the cation.

**6.** The process as claimed in one or more of claims 1 to 5, wherein A in the general formula of the quaternary ammonium ion is an alkyl radical having 10 to 18, in particular 12 to 16, carbon atoms.

**7.** The process as claimed in one or more of claims 1 to 6, wherein B, C and D in the general formula of the quaternary ammonium ion are each a methyl and/or ethyl radical.

**8.** The process as claimed in claim 6 and 7, wherein the quaternary ammonium ion is trimethyldodecylammonium, trimethyltetradecylammonium or trimethylhexadecylammonium.

**9.** The process as claimed in one or more of claims 1 to 8, wherein the aqueous catalyst solution containing rhodium and arylphosphanes has a pH of 5 to 7.

**10.** The process as claimed in one or more of claims 1 to 9, wherein the olefins contain 2 to 24, in particular 8 to 20, preferably 12 to 18, carbon atoms.

**Revendications**

**1.** Procédé préparation d'amines par réaction d'oléfines avec l'oxyde de carbone, l'hydrogène et une amine primaire et/ou secondaire en phase liquide en présent de rhodium et d'arylphosphanes à température élevée et sous pression, caractérisé en ce que l'on utilise en tant qu'arylphosphanes, en solution dans l'eau, des sels de formule générale

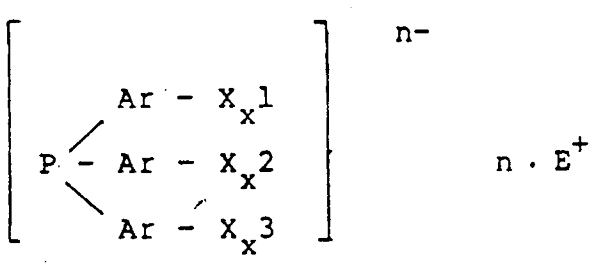

dans laquelle Ar représente un groupe aryle et X un groupe acide sulfonique, $x^1$, $x^2$, $x^3$ sont égaux à 0 ou 1, sous réserve que l'un au moins des indices $x^1$, $x^2$ ou $x^3$ est égal à 1, E représente un atome de métal alcalin, $NH_4^+$ ou un ion ammonium quaternaire de formule générale

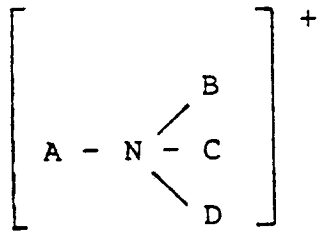

dans laquelle A représente un groupe alkyle en $C_6$-$C_{20}$ et B, C, D des groupes alkyles à chaîne droite ou ramifiée en $C_1$-$C_4$, et n est un nombre entier allant de 1 à 3.

2. Procédé selon revendication 1, caractérisé en ce que la réaction est effectuée à des températures de 100 à 160, plus spécialement de 110 à 150, de préférence de 120 à 140 °C et sous des pressions de 4 à 20, plus spécialement de 8 à 18 et de préférence de 10 à 16 MPa.

3. Procédé selon revendication 1 ou 2, caractérisé en ce que les arylphosphanes utilisés consistent en un mélange de sels alcalins ou d'ammonium et de sels d'ammonium quaternaire de formule générale

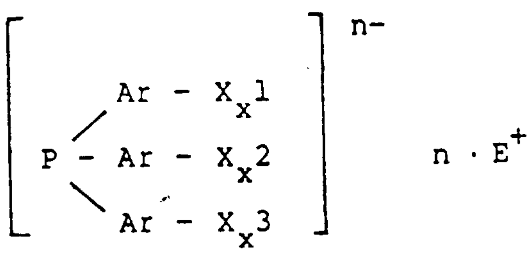

4. Procédé selon une ou plusieurs des revendications 1 à 3, caractérisé en ce que le mélange des arylphosphanes contient de 1 à 40 plus spécialement de 10 à 35 et de préférence de 15 à 33 mol% (par rapport au mélange de phosphanes) de sels d'ammonium quaternaire.

5. Procédé selon une ou plusieurs des revendications 1 à 4, caractérisé en ce que le cation des sels alcalins des arylphosphanes consiste en sodium et/ou potassium.

6. Procédé selon une ou plusieurs des revendications 1 à 5, caractérisé en ce que, dans la formule générale de l'ion ammonium quaternaire, A représente un groupe alkyle en $C_{10}$-$C_{18}$, plus spécialement en $C_{12}$-$C_{16}$.

7. Procédé selon une ou plusieurs des revendications 1 à 6, caractérisé en ce que, dans la formule générale de l'ion ammonium quaternaire, B, C et D représentent chacun un groupe méthyle ou éthyle.

8. Procédé selon les revendications 6 et 7, caractérisé en ce que l'ion ammonium quaternaire est l'ion triméthyldodécylammonium, l'ion triméthyltétradécylammonium ou l'ion triméthylhexadécylammonium.

9. Procédé selon une ou plusieurs des revendications 1 à 8, caractérisé en ce que la solution aqueuse contenant le rhodium et les arylphosphanes présente un pH de 5 à 7.

10. Procédé selon une ou plusieurs des revendications 1 à 9, caractérisé en ce que les oléfines contiennent 2 à 24 plus spécialement 8 à 20 et de préférence 12 à 18 atomes de carbone.